# EUROPÄISCHE PATENTSCHRIFT

(11) **EP 2 397 838 B1**
(45) Veröffentlichungstag und Bekanntmachung des Hinweises auf die Patenterteilung: **17.01.2018**
(21) Anmeldenummer: 10006204.1
(22) Anmeldetag: 15.06.2010
(51) Int. Cl.: G01N 21/39, G01N 33/00, G01N 21/03, G01N 21/15, G01N 21/31, G01N 21/3504

(54) **Gassensor zur Messung von Feuchtigkeit und Kohlendioxid-Konzentration**
Gas sensor for measuring humidity and the concentration of carbon dioxide
Capteur de gaz pour mesurer l'humidité et la concentration de dioxide de carbone

(43) Veröffentlichungstag der Anmeldung: 21.12.2011
(73) Patentinhaber: Axetris AG, 6056 Kägiswil (CH)
(72) Erfinder: Willing, Bert, 1807 Bloney (CH); Kohli, Markus, 1422 Grandson (CH); Seifert, Andreas, 1153 Denens (CH)
(74) Vertreter: Klocke, Peter

(56) Entgegenhaltungen:
- EP-A1- 1 816 464
- WO-A2-03/014713
- DE-A1- 10 220 668
- US-A1- 2007 069 131
- US-A1- 2009 028 209
- US-A1- 2009 231 588
- US-A1- 2010 002 235
- BARBU T L ET AL: "TDLAS a laser diode sensor for the in situ monitoring of H2O, CO2 and their isotopes in the Martian atmosphere" ADVANCES IN SPACE RESEARCH, PERGAMON, OXFORD, GB LNKD- DOI:10.1016/J.ASR.2005.04.049, Bd. 38, Nr. 4, 1. Januar 2006 (2006-01-01) , Seiten 718-725, XP024914659 ISSN: 0273-1177 [gefunden am 2006-01-01]

## Beschreibung

Die vorliegende Erfindung betrifft einen TDLS (Tunable Diode Laser Spectrometry)-Gassensor zumindest zur Messung von Kohlendioxid (CO₂) und ein Verfahren zumindest zur Messung der Feuchtigkeit (H₂O) und des Kohlendioxids (CO₂) in einem Inkubator oder ähnlich aufgebauten Klimakammern. Die Erfindung betrifft insbesondere einen TDLS-Gassensor mit einer Messaufnahme mit opto-elektronischen Komponenten, die mindestens eine Laserdiode als Emitter und einen Detektor für einen von der Laserdiode emittierten Laserstrahl umfasst, und mit einer Absorptionsaufnahme, die ein Volumen aufweist, in dem das zu messende Gas aus einem Innenraum des Inkubators/Klimakammer zirkulieren kann und in dem sich der Laserstrahl ausbreitet, und mit mindestens einem Reflektor, der das von der Laserdiode ausgestrahlte Laserlicht auf den Detektor lenkt.

Ein derartig aufgebauter Gassensor ist aus der DE 102 20 668 A1 bekannt.

Das TDLS-Messprinzip ist allgemein bekannt und ist eine höchst selektive und vielseitige Technik, um viele Spuren atmosphärischer Substanzen mit einer Detektionsempfindlichkeit im ppb-Bereich zu messen. Hierbei wird aus einer gemessenen Absorption die Konzentration des zu untersuchenden Gases bzw. Gasbestandteils bestimmt. Als Strahlungsquelle wird eine Laserdiode verwendet.

Das TDLS-Messprinzip ist beispielsweise in der Offenlegungsschrift EP 1 816 464 A1 beschrieben. Diese Schrift behandelt eine Anordnung zur Konzentrationsmessung für Abgaskomponenten im Abgasbereich einer Feuerungsanlage. Die dort vorgeschlagene Anordnung sowie das zugehörige Verfahren sind insbesondere zur Messung des Sauerstoffgehaltes des Abgases vorgesehen. Die Messanordnung umfasst eine monochrom über Wellenlänge durchstimmbare Lichtquelle, wie beispielsweise eine Laserdiode, eine Absorptionsstrecke, in der sich das zu untersuchende Gas befindet und einen Lichtempfänger, wie beispielsweise eine Photodiode. Zur Detektion wird die TDLS durchgeführt. Die Geometrie der Messanordnung erlaubt es Gaskonzentrationen mit der TDLS an Messorten zu erfassen, an denen mit Verschmutzung bzw. Kondensation von Flüssigkeiten an den Fenstern der Messzelle zu rechnen ist. In der Anordnung kommt ein einfach gefalteter Strahlengang zur Anwendung, wobei die Reflexion des von der Lichtquelle emittierten Lichtes an einer diffus reflektierenden Oberfläche erfolgt. Eine äußerst positive Eigenschaft dieser Anordnung liegt in der geringen Anfälligkeit der Konzeption gegenüber optischen Indifferenzen.

Für biologische Anwendungen werden u.a. Zellenstämme in sogenannten Zell-Inkubatoren gezüchtet. Der Innenraum des Inkubators wird dabei auf einer definierten Atmosphäre (CO₂-Gehalt, Sauerstoffgehalt, Feuchtigkeit) und einer definierten Temperatur gehalten und die entsprechenden Parameter über Sensoren geregelt. Nach dem Entfernen der Zellkulturen wird der Inkubator vor dem nächsten Betriebszyklus im Allgemeinen zunächst sterilisiert. Dies erfolgt entweder bei einer relativen Feuchtigkeit von 100 % und einer Temperatur von 90 °C oder aber in zunehmender Weise bei einer relativen Feuchtigkeit von deutlich unter 100 % und einer Temperatur bis zu 200 °C, jeweils über mehrere Stunden. Derzeitige CO₂- und auch Feuchtigkeitssensoren sind jedoch so aufgebaut, dass sie eine Temperatur von 200 °C auch im ausgeschalteten Zustand nicht überdauern, da sie sich im Innenraum des Inkubators befinden und die entsprechenden elektronischen Bauteile zur Signalverarbeitung eine deutlich niedrigere maximale Lagertemperatur aufweisen. Die Sensoren müssen deshalb vor einer solchen Sterilisierung demontiert werden, was zum Einen Arbeitsaufwand für das nicht mit Sensorik vertraute Personal bedeutet, zum Anderen aber auch die Sensoren in einem undefinierten Sterilisierungszustand belässt. Als CO₂-Sensoren werden entweder sehr ungenaue Temperaturleitfähigkeitssensoren oder NDIR (Non Diffractive lnfrared)-Sensoren verwendet, die sich aufgrund ihres Aufbaus zwingend innerhalb des Inkubators befinden müssen.

Zum Stand der Technik wird weiterhin auf den Fachartikel von Barbu: "TDLAS a laser diode sensor for the in situ monitoring of H2O, CO2 and their isotopes in the Martian atmosphere", Advances in Space Research, Pergamon, Oxford, GB LNKD- DOI:10.1016/J.ASR.2005.04.049, Band 38, Nr. 4, 1. Januar 2006, Seiten 718 - 725, und die Druckschrift US 2009/028 209 A1 verwiesen.

Barbu et al. offenbaren einen TDSL-Gassensor zur gleichzeitigen Messung der CO₂- und der H₂O-Konzentration in einer Gasatmosphäre mittels eines an sich bekannten Absorptions-Spektroskopie-Verfahrens. Die Messanordnung umfasst eine monochrom über die Wellenlänge durchstimmbare Lichtquelle in Form einer Laserdiode, eine Absorptionsstrecke, in der sich das zu untersuchende Gas befindet und einen Lichtempfänger, wie beispielsweise eine Fotodiode. Als Lichtquelle wird eine DFB GalnSb-Laserdiode verwendet, die einen Lichtstrahl der Wellenlänge in der Größenordnung von 1877 nm durch die Absorptionsstrecke auf den Fotodiode richtet.

Die US 2009/028 209 A1 lehrt ein Sensor-System zur Messung von Oxidationsprodukten in einer Gasatmosphäre. Das System weist eine Lichtquelle auf, die einen Lichtstrahl durch eine Oxidationskammer mit einem Probevolumen auf den Lichtdetektor richtet. Eine Auswerteeinheit mit mit einem Mikroprozessor quantifiziert die Konzentration der Oxidationsprodukte in dem Probevolumen. Die Lichtquelle und der Detektor sind in einem Spektrometergehäuse angeordnet, durch das das Probevolumen von einem Eingang zu einem Ausgang strömt. Mittels des Sensor-Systems kann beispielsweise die Konzentration von Kohlenmonoxid, Kohlendioxid oder Wasserdampf in der Gasatmosphäre ermittelt werden.

Die prinzipiellen Schwierigkeiten eines Sensors für Messungen in einem Inkubator oder einer ähnlich aufgebauten Klimakammer liegen in dem Temperaturprofil, das in dem Gassensor herrscht, da der Inkubator bei 37 °C betrieben und bei höheren Temperaturen bis zu 200 °C sterilisiert wird, und Laser sowie Fotodioden nicht wärmer als ca. 80 °C werden dürfen. Ein Problem beim Messen stellen außerdem Rückreflektionen in die Laserdiode dar, die einen instabilen Zustand bewirken können.

Der vorliegenden Erfindung liegt daher die Aufgabe zugrunde, eine Möglichkeit vorzuschlagen, die zumindest eine genaue und von Reflektionen ungestörte Messung von CO₂ erlaubt und mit der das zu untersuchende Gas umfassender analysiert werden kann, wobei der Gassensor auch beim Sterilisieren im eingebauten Zustand verbleiben kann. Der vorliegenden Erfindung liegt außerdem die Aufgabe zugrunde, ein Verfahren zur gleichzeitigen Messung von Kohlendioxid und Feuchtigkeit vorzuschlagen.

Diese Aufgabe wird erfindungsgemäß durch einen TDLS-Gassensor mit den Merkmalen des Anspruchs 1 gelöst. Weitere vorteilhafte Ausgestaltungen sind den jeweils rückbezogenen Unteransprüchen zu entnehmen.

Gemäß der Erfindung wird ein auf dem TDLS-Messprinzip basierender Gassensor der eingangs charakterisierten Art verwendet, der ein Fenster zur Trennung der Laserdiode von dem Innenraum und damit der Atmosphäre des Inkubators aufweist, wobei das Fenster schräg zu der Achse des von der Laserdiode emittierten Laserstrahls angeordnet ist. Des weiteren weist der TDLS-Gassensor vorzugsweise einen als Wärmesenke dienenden Materialblock aus thermisch gut leitendem Material auf, der in der Messaufnahme angeordnet ist und die opto-elektronischen Komponenten, insbesondere Laserdiode und Detektor aufnimmt. Des weiteren weist der TDLS-Gassensor eine Heizeinrichtung für das Fenster auf, die eine Kondensation an dem Fenster durch Beheizen verhindert. Die den TDLS-Gassensor bildende Einheit aus Messaufnahme und Absorptionsaufnahme kann entweder vor dem Einbau in die Öffnung in der Wand des Inkubators oder erst nach dem Einstecken der Messaufnahme in die Wand des Inkubators hergestellt werden. Die Wand kann eine beliebige Wand des Inkubators sein, beispielsweise eine Seitenwand, ein Boden oder ein Deckel. Diese unterschiedlichen Ausgestaltungen werden unter anderem dadurch bedingt, ob der Laser mit einem divergierenden Strahl betrieben wird, so dass bei der entsprechend erforderlichen Absorptionsstrecke der Spiegel zur Rückreflektierung und -fokussierung einen zu großen Durchmesser hat, als dass er von außen in den Inkubator eingesteckt werden kann. Hierbei ist die Messaufnahme in eine Öffnung in der Wand des Inkubators einsteckbar und mit der Absorptionsaufnahme im Innenraum des Inkubators zur optischen Ausrichtung von Laserdiode, Reflektor und Detektor verbindbar. Bei der Verwendung eines kollimierten Laserstrahls kann bei einem deutlich verkleinerten Durchmesser der TDLS-Gassensor komplett von außen in die Wand des Inkubators eingesteckt werden. Mit der erfindungsgemäßen Ausbildung des TDLS-Gassensors wird eine betriebssichere und genaue Messung ermöglicht. Das schräg zur Achse der Laserdiode angeordnete Fenster, das beispielsweise aus einem kristallinen oder amorphen für den Laserstrahl durchlässigen Glasmaterial oder einem anderen geeigneten Festkörper gebildet sein kann, verhindert Rückreflektionen, die die Messung beeinträchtigen könnten. Darüber hinaus dient das Fenster der Trennung der unterschiedlichen Atmosphären, da Laser- und eine vorzugsweise als Detektor verwendete Fotodiode nicht wärmer als 60 bis 65 °C werden dürfen. Als Fenster kann auch ein Keilfenster, welches als spezielle Ausführungsform eines schräg zur Achse der Laserdiode angeordnetes Fenster angesehen werden kann, eingesetzt werden. Um den Wärmeabbau bei höheren Temperaturen im Inkubator zu gewährleisten, sitzen die opto-elektronischen Komponenten wie Laser- und Fotodiode vorzugsweise in einem Materialblock aus thermisch gut leitendem Material, vorzugsweise in einem Metallblock beispielsweise aus Aluminium, der als Wärmesenke an die Umgebungstemperatur dient. Erfindungsgemäß sind die opto-elektronischen Komponenten in der Messaufnahme von der Absorptionsaufnahme thermisch entkoppelt, indem die Absorptionsaufnahme und die Messaufnahme über einen rohrförmigen Abschnitt aus temperaturfestem Kunststoffmaterial miteinander verbunden sind. Des weiteren sorgt eine Heizeinrichtung für das Fenster, dass bei normalem Betrieb keine Kondensation auf dem Fenster erfolgt. Diese entsteht ohne Heizung dadurch, dass bei normalem Betrieb des Inkubators mit einer relativen Luftfeuchtigkeit von nahezu 100% im Innenraur des Inkubators und der Wärmeabfuhr, die durch das Fenster sowie den Materialblock erfolgt, das Fenster auf einer tieferen Temperatur als der Inkubatorinnenraum liegt. Bei der Heizeinrichtung kann es sich um eine Beschichtung, einen IR-Strahler, einen Lastwiderstand oder ähnliche geeignete Einrichtungen handeln, die sicherstellen, dass die von der Heizeinrichtung erzeugte Wärme bei normalem Betrieb des Inkubators eine Kondensation an dem Fenster verhindern. Im Sterilisationsbetrieb wird diese Heizeinrichtung ausgeschaltet. Erfindungsgemäß misst die Ansteuerungs- und Auswerteelektronik mittels der Laserdiode zusätzlich die Feuchtigkeit, vorzugsweise bei einer Wellenlänge im Bereich von 1950 bis 1960 nm, vorzugsweise 1953 nm. Es kann somit mit dem TDLS-Gassensor sowohl CO₂ als auch H₂O mit einer Einrichtung in einem Messzyklus, zum Beispiel 10 Sekunden CO₂ und 10 Sekunden Feuchtigkeit, gemessen werden, da die Wellenlänge der entsprechenden Absorptionslinien innerhalb des Durchstimmbereichs des verwendeten Diodenlasers liegen. Der Messzyklus kann dazu aus einem einzigen Abfahren des ganzen Durchstimmbereichs bestehen oder aus alternierendem Abfahren von zwei Teilbereichen, die den Absorptionswellenlängen von CO₂ und H₂O entsprechen. Dabei können die beiden Messungen parallel oder seriell erfolgen.

Vorteilhafterweise werden mittels der Ansteuerungs- und Auswerteelektronik die benachbarten CO₂- und H₂O-Absorptionslinien bei 1953 nm gemessen und damit die Querempfindlichkeit zwischen CO₂ und H₂O kompensiert.

Gemäß einer weiteren Ausbildung der Erfindung weist die Messaufnahme eine weitere Laserdiode und eine weitere Fotodiode für eine Sauerstoff (O₂)- Messung und Auswertung mittels der Ansteuerungs- und Auswerteelektronik auf.

Die Messaufnahme und die Absorptionsaufnahme können von der Gestaltung äußerlich sowohl quaderförmig als auch zylinderförmig ausgebildet sein. Wesentlich ist, dass die optische Ausrichtung der für die Messung notwendigen Elemente wie Laserdiode, Reflektor und Detektor für die Messung geeignet ist und sich beim Zusammenbau nicht verändert. Das Fenster soll die Laserstrahlen ungehindert durchlassen und besteht daher vorzugsweise aus Glas, das möglichst eine hohe Widerstandsfähigkeit gegen Glasfraß aufweist. Grundsätzlich kann das Fenster an der Messaufnahme oder an der Absorptionsaufnahme angeordnet sein. Der Strahl tritt durch das Fenster in den Inkubator ein, wobei der Reflektor so angeordnet ist, dass er sich in einer vorgebbaren Entfernung von der Wand des Inkubators befindet. Durch die Öffnungen in den Wänden der Absorptionsaufnahme und/oder dem Reflektor gelangt das zu untersuchende Gas in den Bereich zwischen Reflektor und Fenster. Gemäß einer vorteilhaften Ausgestaltung ist der TDLS-Gassensor als teilbare Einheit aus Messaufnahme und Absorptionsaufnahme ausgebildet. Die Verbindung der Messaufnahme mit der im Innenraum angeordneten Absorptionsaufnahme kann mittels eines Gewindes oder eines Bajonnetverschlusses erfolgen. Der TDLS-Sensor dichtet im Bereich der Absorptionsaufnahme gegenüber der Wand des Inkubators ab.

Gemäß einer bevorzugten Ausbildungsform weist der TDLS-Gassensor im Bereich der Messaufnahme einen rohrförmigen Abschnitt aus einem temperaturfesten Kunststoff auf, mit dem er in die Öffnung in der Wand des Inkubators einsteckbar ist. Die Laserdiode und der Detektor, vorzugsweise eine Fotodiode, werden mit einem definierten Abstand in dem massiven Materialblock aus thermisch gut leitendem Material, vorzugsweise aus Aluminium angeordnet. Der rohrförmige Abschnitt ist aus einem temperaturfesten Kunststoff, beispielsweise PPS, um eine Wärmeleitung von der Wand des Inkubators zu den elektronischen Bauteilen zu verhindern und diese zu schützen. Der Abstand der Laserdiode wird dadurch bestimmt, dass bei einer Betriebstemperatur des Inkubators von 200 °C zur Sterilisierung die Temperatur von 180 °C am Fenster auf unter 65 °C an der Laserdiode abfallen muss.

In einer weiteren Ausbildung der Erfindung ist der rohrförmige Abschnitt zumindest im dem Bereich des Materialblockes mit der Laserdiode und dem Detektor, vorzugsweise einer Fotodiode innen gegenüber der Absorptionsaufnahme zentriert, beispielsweise mittels eines Konus, wobei eine Druckfeder auf der der Laserdiode gegenüberliegenden Rückseite des Materialblock aus thermisch gut leitendem Materials diesen in den rohrförmigen Abschnitt oder auch an der Absorptionsaufnahme fixiert. Vorzugsweise wird durch die Feder ein am Materialblock befindlicher Konus in eine entsprechende Gegenfläche an der Absorptionsaufnahme gepresst, so dass die Laserdiode und die Fotodiode in der Messaufnahme und der Reflektor in der Absorptionsaufnahme in einer eindeutig zueinander definierten Position sitzen.

Die Absorptionsaufnahme ist in einer weiteren Ausbildung der Erfindung als zylindrisches Teil ausgebildet, das am einen Ende den Reflektor und am anderen Ende in einem in die Wand des Inkubators einsteckbaren Rohrabschnitt ein Fenster und mit länglichen Ausnehmungen versehene Umfangswände aufweist. Die Ausgestaltung der Absorptionsaufnahme als ein zylindrisches Teil hat fertigungstechnische Vorteile. Die Absorptionsaufnahme schließt gegen die Messaufnahme mit einem Fenster gasdicht ab und hält beabstandet von der Wand des Inkubators den Reflektor, der gemäß einer weiteren bevorzugten Ausbildung als Konvexspiegel, vorzugsweise aus elektrochemisch poliertem Edelstahl ausgebildet ist.

Elektrochemisch poliertes Edelstahl wird bevorzugt, da bedampfte Spiegel in sehr heißen und feuchten Atmosphären sehr schnell altern. Die Absorptionsaufnahme ist aus einem hoch temperaturfesten Kunststoff, beispielsweise PPS hergestellt. Gemäß einer weiteren Ausbildung ist der Detektor, vorzugsweise eine Fotodiode nicht genau im Fokus des Konvexspiegels angeordnet, sondern etwas davor bzw. dahinter, damit mögliche Justierungstoleranzen kein Auswandern des Strahls erzeugen.

Verfahrensgemäß wird die Messung Feuchtigkeit und Kohlendioxid in den Innenraum eines Inkubators oder ähnlich aufgebauter Klimakammer nach dem TDLS-Prinzip mittels des erfindungsgemäß ausgestatteten Sensors durchgeführt, in dem die absolute Feuchtigkeit und die Kohlenstoffdioxid-Konzentration in einem Messzyklus gemessen werden. Hierbei wird vorzugsweise die Laserdiode bei einer Wellenlänge von 1950 bis 1960 betrieben. Damit werden die benachbarten Kohlendioxid und Wasser-Absorptionslinien bei 1953 nm gemessen, so dass die Querempfindlichkeiten zwischen Kohlendioxid und Feuchtigkeit durch benachbarte Kohlendioxid- und Wasser-Absorptionslinien kompensierbar sind. Vorzugsweise erfolgt zusätzlich eine Sauerstoffmessung mittels einer weiteren Diode in einem dafür geeigneten Wellenlängenbereich von 760 nm. Die Heizeinrichtung für das Fenster wird vorteilhafterweise nur im Normalbetrieb eingeschaltet. Die gerichtete Strahlung des Lasers zusammen mit einer Wellenlänge, für die normales Fensterglas durchlässig ist, ermöglicht deshalb einen Messaufbau, bei dem die gesamten elektronischen Bauelemente (Laserdiode, Detektor, vorzugsweise Fotodiode, Ansteuer- und Auswerteelektronik) außerhalb des Inkubators liegen und sich auf Raumtemperatur befinden. Durch die Auslegung des Sensors befindet sich das in der Absorptionsaufnahme angeordnete Fenster auf der gleichen Temperatur wie der Innenraum des Inkubators. Bei einer nur leicht
geringeren Temperatur als die Temperatur des Inkubators käme es durch die hohe Feuchtigkeit im Inkubator zu einer Kondensation auf dem Fenster, wodurch der Betrieb des Sensors beeinträchtigt würde. Der Laserstrahl wird durch das Fenster in den Innenraum des Inkubators geschickt, dort von dem Spiegel reflektiert und läuft wiederum durch das Fenster auf die Fotodiode. Fenster, Spiegel und Spiegelhalter sind kostengünstig aus Materialien zu fertigen, die die verwendeten Sterilisationsmöglichkeiten langfristig ohne Beeinträchtigung verkraften. Damit muss der Sensor während des Sterilisationszyklusses nicht demontiert werden und wird mitsterilisiert. Dadurch, dass der Gassensor bevorzugt aus zwei leicht zu trennenden Bauteilen, nämlich der Messaufnahme und der Absorptionsaufnahme besteht, können Verschmutzungen (Staub, Kondensation, Zellreste, Tropfen von Lösungsmitteln) leicht durch Reinigen entfernt werden. Zudem ist die Absorptionsaufnahme ein Bauteil, das vom Endbenutzer des Inkubators ohne spezielles Wissen leicht ausgetauscht werden kann, ohne dass teure Bauteile mit getauscht werden müssen.

Nachfolgend wird die Erfindung an Hand eines in der Zeichnung dargestellten Ausführungsbeispiels näher erläutert. Es stellen dar:
- Figur 1: einen Längsschnitt durch einen TDLS-Gassensor mit einer Messaufnahme und einer Absorptionsaufnahme, wobei die Absorptionsaufnahme von dem Innenraum des Inkubators mit der in einer Öffnung des Inkubators eingesteckten Messaufnahme verbindbar ist;
- Figur 2: den Längsschnitt durch einen TDLS-Gassensor in einer prinzipiellen Darstellung mit einer zusätzlichen Laserdiode für die Sauerstoffmessung und bei dem der gesamte TDLS-Gassensor in einer Öffnung in der Inkubatorwand einsteckbar ist, und
- Figur 3: einen Querschnitt durch den TDLS-Gassensor gemäß Figur 2 entlang der Linie A-A.

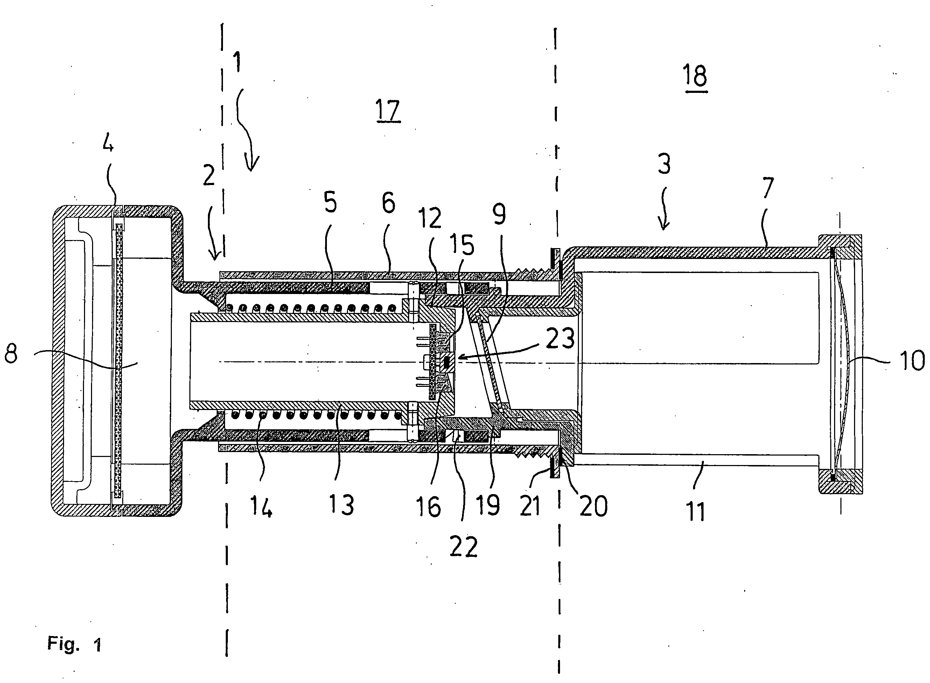

Die Figur 1 zeigt den Längsschnitt durch den TDLS-Gassensor 1 in einer rohrförmigen Durchführung 6, die in einer nicht dargestellten, mit 17 angedeuteten Wand eines Inkubators angeordnet ist. Der TDLS-Gassensor 1 weist eine Messaufnahme 2 und einer Absorptionsaufnahme 3 auf. Die Messaufnahme 2 befindet sich außerhalb eines nicht dargestellten mit 18 angedeuteten Innenraums des Inkubators teilweise in der Wand 17 des Inkubators. Je nach Aufbau des Inkubators kann die Wand 17 aus Doppelwänden mit dazwischenliegenden Heizeinrichtungen oder aus einer Einfachwand mit außen aufgebrachter Isolation bestehen. Die Messaufnahme 2 weist ein quaderförmigen Gehäuseteil 4 und ein daran anschließendes rohrförmiges Gehäuseteil 5 auf. In dem Gehäuseteil 4 befindet sich die Ansteuerungs- und Auswerteelektronik 8. In dem rohrförmigen Gehäuseteil 5 befinden sich in einem zylindrischen Aluminiumblock 13 eine Laserdiode 15 und eine Fotodiode 16. Der Aluminiumblock 13 dient als Wärmesenke. Er kann bei Bedarf auch massiv ausgeführt sein, wobei am Ende ein Kopf mit Laserdiode 15 und Fotodiode 16 befestigt sein kann. An seiner die Laserdiode 15 und die Fotodiode 16 aufnehmenden Stirnseite ist der Aluminiumblock 13 am Umfang konisch ausgebildet, um als Zentrierung 12 für den Aluminiumblock 13 in einem angepassten Rohrabschnitt 19 der Absorptionsaufnahme 3 zu dienen. Der Aluminiumblock 13 wird mittels einer Feder 14, die sich in dem Ausführungsbeispiel an dem Gehäuseteil 4 abstützt, mit der konischen Zentrierung 12 in den Rohrabschnitt 19 gedrückt und sorgt für die zentrische Lage der Laserdiode 15 und der Fotodiode 16.

Die Absorptionsaufnahme 3 weist ein zylindrisches Gehäuse 7 mit Ausnehmungen 11 im Anschluss an den Rohrabschnitt 19 auf, durch das zu messende Gas hindurchtreten kann. Somit befindet sich die Absorptionsaufnahme 3 mit ihrer Messtrecke in dem Innenraum 18 des Inkubators und der in der Wand 17 befindliche Rohrabschnitt 19 dient der Lagerung in der Wand 17, der Fixierung an der Messaufnahme 2 und der optischen Ausrichtung. Die Abdichtung zu der Wand 17 des Inkubators erfolgt mit einer Dichtung 20 zwischen dem Gehäuse 7 der Absorptionsaufnahme 3 und der Durchführung 6 und einer Dichtung 21 zwischen der Durchführung 6 und der Wand 17. In dem Rohrabschnitt 19 befindet sich ein geneigtes planparalleles Glasfenster 9, das den Innenraum 18 des Inkubators gegenüber der Messaufnahme 2 abdichtet, damit die Funktion der Laserdiode 15 nicht beeinträchtigt wird. An dem dem Fenster 9 gegenüberliegenden Ende der Absorptionsaufnahme 3 befindet sich in dem Gehäuse 7 ein Konvexspiegel 10 aus elektrochemisch poliertem Edelstahl. Die Messaufnahme 2 wird in die Durchführung 6 eingesteckt und von innen mit der Absorptionsaufnahme 3 mittels eines Bajonettverschlusses 22 fixiert. Zweckmäßigerweise wird zuvor das Gehäuseteil 4 und damit die Messaufnahme 2 mittels geeigneten Einrichtungen, beispielsweise durch Anschrauben, an der Wand 17 oder in der Durchführung 6 befestigt, da normalerweise von einer Person nicht die Messaufnahme 2 und die Absorptionsaufnahme 3 gleichzeitig gehalten werden können. Durch den zentrischen Aufbau wird gewährleistet, dass die optischen Elemente beim Zusammenbau aufeinander ausgerichtet bleiben.

Der Aluminiumblock 13 ist von dem Fenster 9 beabstandet, damit die Laserdiode 15 und die Fotodiode 16 der beim Sterilisieren in dem Inkubator auftretenden hohen Temperatur nicht ausgesetzt werden. Der dadurch entstehende Totraum der gesamten Messstrecke von Laserdiode 15 zu Spiegel 10, der nicht dem zu messenden Gas ausgesetzt ist, muss bei der Auswertung des Messsignals berücksichtigt werden. Die tatsächliche Absorptionsstrecke ist der Bereich zwischen dem Fenster 9 und dem Spiegel 10. Der Abstand zwischen Fenster 9 und Laserdiode 15 liegt in der Größenordnung von 8 bis 10 mm, wobei, falls erforderlich, der Rohrabschnitt 19 gegenüber der Darstellung in der Figur noch verkürzt werden kann, um den Abstand zu vergrößern. Zwischen der Fotodiode 16 und der Laserdiode 15 befindet sich in dem Ausführungsbeispiel ein Heizwiderstand 23, der für die Erwärmung des Fensters 9 im Normalbetrieb sorgt.

Die Laserdiode 15 muss bei einer geforderten Messgenauigkeit von circa 2000 ppm CO₂ nicht kollimiert betrieben werden. Der Strahl tritt durch das geneigte, planparallele Fenster 9 in den Innenraum des Inkubators ein und wird in einer Entfernung von circa 10 cm von dem Konvexspiegel 10 mit einem Durchmesser von 50 mm zurück durch das Fenster 9 auf die Fotodiode 16 fokussiert, wobei die Fotodiode nicht genau im Fokus des Spiegels 10, sondern etwas davor bzw. dahinter liegt. Das Fenster 9 ist geneigt, um Rückreflektionen auf die Laserdiode 15 zu vermeiden bzw. zu reduzieren.

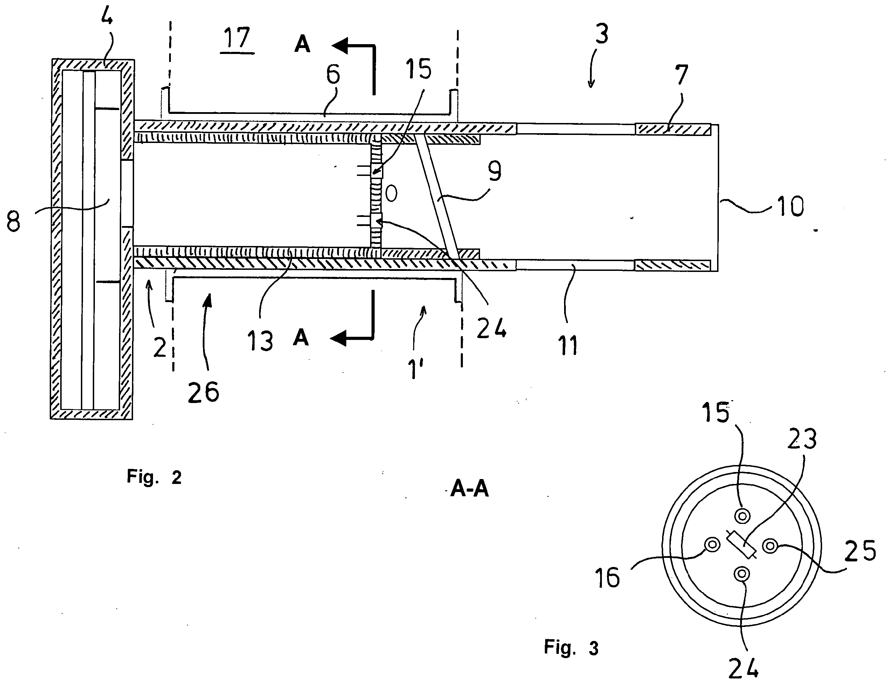

Figur 2 zeigt schematisch den Längsschnitt durch einen TDLS-Gassensor 1', der einen geringeren Durchmesser hat und somit als ganze Einheit mit einem rohrförmigen Gehäuseteil 26 durch die Durchführung 6 gesteckt werden kann. Bei diesem Ausführungsbeispiel sind zusätzlich eine weitere Laserdiode 24 und eine weitere Fotodiode 25 vorgesehen, um mit diesem Gassensor auch eine Sauerstoffmessung durchführen zu können. Die übrigen Teile sind entsprechend dem TDLS-Gassensor 1 gemäß Figur 1 ausgebildet. Selbstverständlich kann auch der Gassensor 1 gemäß Figur 1 mit einer zusätzlichen Laserdiode für die Sauerstoffmessung versehen sein bzw. der Aufbau gemäß Figur 2 ohne zusätzliche Laserdiode 24 und Fotodiode 25 ausgebildet sein.

## Patentansprüche

1. TDLS-Gassensor (1, 1') zur Messung der Kohlendioxid-Konzentration in einem Inkubator oder in einer ähnlich aufgebauten Klimakammer, umfassend:
eine Messaufnahme (2) mit opto-elektronischen Komponenten, die eine Ansteuerungs- und Auswerteelektronik (8), eine Laserdiode (15) als Emitter und einen Detektor (16) für einen von der Laserdiode (15) emittierten Laserstrahl umfasst,
eine Absorptionsaufnahme (3), die ein Volumen aufweist, in dem das zu messende Gas aus einem Innenraum des Inkubators, oder der ähnlich aufgebauten Klimakammer, zirkulieren kann und in dem sich der Laserstrahl ausbreitet, und mit mindestens einem Reflektor (10), der das von der Laserdiode (15) ausgestrahlte Laserlicht auf den Detektor (16) lenkt,
wobei die Messaufnahme (2) und die Absorptionsaufnahme (3) durch ein Fenster (9) atmosphärisch abgetrennt sind und die opto-elektronischen Komponenten in der Messaufnahme (2) von der Absorptionsaufnahme (3) thermisch entkoppelt sind, indem die Absorptionsaufnahme (3) und die Messaufnahme (2) über einen rohrförmigen Abschnitt aus temperaturfestem Kunststoffmaterial miteinander verbunden sind, und wobei die Ansteuerungs- und Auswerteelektronik (8), Laserdiode (15) und Detektor (16) ausgelegt sind für die Messung der Feuchtigkeit und der Kohlenstoffdioxid-Konzentration, wobei die Ansteuerungselektronik vorzugsweise zum Betreiben der Laserdiode (15) bei einer Wellenlänge von 1950 bis 1960 nm ausgelegt ist, wobei das Fenster (9) ein schräg zu der Achse des von der Laserdiode (15) emittierten Laserstrahls angeordnetes Fenster (9) ist, und eine Heizeinrichtung (23) für das Fenster (9), die eine Kondensation an dem Fenster (9) durch Beheizen verhindert.

2. TDLS-Gassensor nach Anspruch 1, wobei die Messaufnahme (2) einen als Wärmesenke dienenden Materialblock aus thermisch gut leitendem Material (13), vorzugsweise einen Metallblock, aufweist, in dem die opto-elektronischen Komponenten angeordnet sind.

3. TDLS-Gassensor nach Anspruch 1 oder 2, wobei die Ansteuerungs- und Auswerteelektronik (8) dazu ausgebildet ist, die Heizeinrichtung (23) für das Fenster (9) im Sterilisationsbetrieb des Inkubators auszuschalten.

4. TDLS-Gassensor nach einem der vorstehenden Ansprüche, wobei die thermische Entkopplung zwischen den opto-elektronischen Komponenten und der Absorptionsaufnahme (3) durch eine mechanische Verbindung des rohrförmigen Abschnitts aus temperaturfestem Kunststoffmaterial von der Messaufnahme (2) zu der Absorptionsaufnahme (3) hergestellt ist.

5. TDLS-Gassensor nach einem der vorangegangenen Ansprüche, wobei die Ansteuerungs- und Auswerteelektronik (8) dazu ausgebildet ist eine Messung der Feuchtigkeit und der Kohlenstoffdioxid-Konzentration in einem gemeinsamen Messzyklus durchzuführen.

6. TDLS-Gassensor nach einem der vorstehenden Ansprüche, wobei die Messaufnahme (2) und die Absorptionsaufnahme (3) lösbar miteinander verbunden sind, wobei die Verbindung der Messaufnahme (2) mit der im Inneren des Inkubators/Klimakammer angeordneten Absorptionsaufnahme (3) mittels eines Gewindes oder eines Bajonettverschlusses erfolgt.

7. TDLS-Gassensor nach den Ansprüchen 2 und 4, wobei die mechanische Verbindung von der Messaufnahme (2) zur der Absorptionsaufnahme (3) den rohrförmigen Abschnitt (5) im Bereich der Messaufnahme (2) aufweist, mit dem sie in die Öffnung in der Wand (17) des Inkubators einsteckbar ist und wobei die Laserdiode (15) und der Detektor (16), vorzugsweise eine Fotodiode (16), von der Wand (17) mit einem definierten Abstand in dem Materialblock (13), vorzugsweise aus Aluminium, angeordnet sind.

8. TDLS-Gassensor nach einem der vorangegangenen Ansprüche 2 bis 7, wobei der Materialblock (13) mit der Laserdiode (15) und dem Detektor (16), vorzugsweise der Fotodiode (16) relativ zu der Absorptionsaufnahme (3) zentriert ist und eine Feder (14) auf der der Laserdiode (15) gegenüberliegenden Rückseite des Materialblocks (13) diesen in eine Zentrierung (12) drückt.

9. TDLS-Gassensor nach einem der vorstehenden Ansprüche, wobei die Absorptionsaufnahme (3) als zylindrisches Teil ausgebildet ist, das an einem Ende den Reflektor (10), am anderen Ende das Fenster (9) und mit Ausnehmungen (11) versehene Umfangswände (7) aufweist.

10. TDLS-Gassensor nach einem der vorangegangenen Ansprüche, wobei der Reflektor (10) als Konvexspiegel, vorzugsweise aus elektrochemisch poliertem Edelstahl, ausgebildet ist, und vorzugsweise der Detektor (16), vorzugsweise eine Fotodiode (16), nicht genau im Fokus des Konvexspiegels (10) angeordnet ist.

11. TDLS-Gassensor nach einem der vorangegangenen Ansprüche, wobei die Messaufnahme (2) eine weitere Laserdiode (24) für eine Sauerstoff Messung und Auswertung mittels der Ansteuerungs- und Auswerteelektronik (8) aufweist.

## Claims

1. TDLS gas sensor (1, 1') for measuring the carbon dioxide concentration in an incubator or in a climate chamber of similar design, comprising:
a measuring pick-up (2) with optronic components that comprises an electronic control and analysis system (8), a laser diode (15) as emitter and a detector (16) for a laser beam emitted by the laser diode (15),
an absorption pick-up (3) that has a volume in which the gas to be measured can circulate from an interior chamber of the incubator or a climate chamber of similar design and in which the laser beam propagates, and with at least one reflector (10) that guides the laser light emitted by the laser diode (15) to the detector (16), in which the measuring pick-up (2) and the absorption pick-up (3) are separated atmospherically by a window (9) and that the optronic components in the measuring pick-up (2) are thermally uncoupled from the absorption pick-up (3) by a connection between the measuring pick-up (2) and the absorption pick-up (3) with a tubular section made of a plastic material of high thermal strength, and
in which the electronic control and analysis system (8), laser diode (15) and detector (16) are designed to measure the moisture and the carbon dioxide concentration, in which the electronic control system is preferable designed to operate the laser diode (15) at a wavelength in the range from 1950 to 1960 nm,
in which the window (9) is arranged oblique with respect to the axis of the laser beam emitted by the laser diode (15), and
a heating system (23) for the window (9) that prevents condensation on the window (9) by heating it.

2. TDLS gas sensor as claimed in claim 1, in which the measuring pick-up (2) comprises a block of material made of thermally well-conducting material (13) that serves as a heat sink, preferably a metal block, in which the optronic components are arranged.

3. TDLS gas sensor as claimed in claim 1 or 2, in which the electronic control and analysis system (8) is designed to deactivate the heating system (23) for the window (9) when the incubator operates in sterilization mode.

4. TDLS gas sensor as claimed in any one of the preceding claims, in which the thermal uncoupling between the optronic components and the absorption pick-up (3) is accomplished by means of a mechanical connection with the tubular section made of a plastic material of high thermal strength from the measuring pick-up (2) to the absorption pick-up (3).

5. TDLS gas sensor as claimed in any one of the preceding claims, in which the electronic control and analysis system (8) is designed to measure the moisture and the carbon dioxide concentration in a combined measuring cycle.

6. TDLS gas sensor as claimed in any of the preceding claims, in which the measuring pick-up (2) and the absorption pick-up (3) form a separable unit, in which the measuring pick-up (2) and the absorption pick-up (3), which is arranged inside the incubator/climate chamber, are connected by means of a thread or a bayonet joint.

7. TDLS gas sensor as claimed in claims 2 and 4, in which the mechanical connection from the measuring pick-up (2) to the absorption pick-up (3) comprises the tubular section (5) in the region of the measuring pick-up (2) with which it can be inserted into the wall (17) of the incubator, and that the laser diode (15) and the detector (16), preferably the photo diode (16), are arranged in the block of material (13), preferably made of aluminum, at a defined distance from the wall (17).

8. TDLS gas sensor as claimed in any one of the preceding claims 2 to 7, in which the block of material (13) with the laser diode (15) and the detector (16), preferably the photo diode (16), is centered relative to the absorption pick-up (3), and that a spring (14) on the rear side of the block of material (13) opposite the laser diode (15) presses said block into a centering device (12).

9. TDLS gas sensor as claimed in any one of the preceding claims, in which the absorption pick-up (3) is shaped as a cylindrical part that has the reflector (10) at one end, the window (9) on the other end and circumferential walls (7) that have recesses (11).

10. TDLS gas sensor as claimed in any one of the preceding claims, in which the reflector (10) consists of a convex mirror, preferably made of electro-chemically polished stainless steel, and that preferably the detector (16), preferably the photo diode (16), is not located exactly in the focal point of the convex mirror (10).

11. TDLS gas sensor as claimed in any one of the preceding claims, in which the measuring pick-up (2) has an additional laser diode (24) for measuring oxygen and for its analysis by means of an electronic control and analysis system (8).

## Revendications

1. Capteur de gaz TDLS (1, 1') pour mesurer la concentration en dioxyde de carbone dans un incubateur ou une chambre climatique de structure analogue, comprenant :
une cellule de mesure (2) contenant des composants optoélectroniques qui comprennent une électronique de commande et d'évaluation (8), une diode laser (15) comme émetteur et un détecteur (16) pour un faisceau laser émis par la diode laser (15),
une cellule d'absorption (3) qui présente un volume dans lequel le gaz à mesurer provenant d'un espace intérieur de l'incubateur ou de la chambre climatique de structure analogue peut circuler et dans laquelle le faisceau laser se propage, et qui comporte au moins un réflecteur (10) qui dévie la lumière laser émise par la diode laser (15) sur le détecteur (16),
dans lequel la cellule de mesure (2) et la cellule d'absorption (3) sont séparées atmosphériquement par une fenêtre (9) et les composants optoélectroniques dans la cellule de mesure (2) sont découplés thermiquement de la cellule d'absorption (3) par le fait que la cellule d'absorption (3) et la cellule de mesure (2) sont reliées entre elles par une section tubulaire en matière plastique résistante à la température, et
dans lequel l'électronique de commande et d'évaluation (8), la diode laser (15) et le détecteur (16) sont prévus ou conçus pour la mesure de l'humidité et de la concentration en dioxyde de carbone, l'électronique de commande étant de préférence prévue ou conçue pour faire fonctionner la diode laser (15) à une longueur d'onde de 1950 à 1960 nm,
dans lequel la fenêtre (9) est une fenêtre (9) disposée obliquement par rapport à l'axe du faisceau laser émis par la diode laser (15) et un dispositif de chauffage (23) pour la fenêtre (9) empêche une condensation sur la fenêtre (9) en la chauffant.

2. Capteur de gaz TDLS selon la revendication 1, dans lequel la cellule de mesure (2) présente un bloc de matière en matériau (13) ayant une bonne conductivité thermique, de préférence un bloc en métal, servant de dissipateur thermique, dans lequel les composants optoélectroniques sont disposés.

3. Capteur de gaz TDLS selon la revendication 1 ou 2, dans lequel l'électronique de commande et d'évaluation (8) est réalisée pour désactiver le dispositif de chauffage (23) pour la fenêtre (9) dans le mode de stérilisation de l'incubateur.

4. Capteur de gaz TDLS selon l'une des revendications précédentes, dans lequel le découplage thermique entre les composants optoélectroniques et la cellule d'absorption (3) est réalisé par une liaison mécanique de la section tubulaire en matière plastique résistante à la température entre la cellule de mesure (2) et la cellule d'absorption (3).

5. Capteur de gaz TDLS selon l'une des revendications précédentes, dans lequel l'électronique de commande et d'évaluation (8) est réalisée pour effectuer une mesure de l'humidité et de la concentration en dioxyde de carbone dans un cycle de mesure commun.

6. Capteur de gaz TDLS selon l'une des revendications précédentes, dans lequel la cellule de mesure (2) et la cellule d'absorption (3) sont reliées entre elles de manière détachable, la liaison de la cellule de mesure (2) avec la cellule d'absorption (3) disposée à l'intérieur de l'incubateur/de la chambre climatique s'effectuant au moyen d'un filetage ou d'un verrouillage à baïonnette.

7. Capteur de gaz TDLS selon les revendications 2 et 4, dans lequel la liaison mécanique entre la cellule de mesure (2) et la cellule d'absorption (3) présente la section tubulaire (5) dans la zone de la cellule de mesure (2), avec laquelle elle peut être introduite dans l'ouverture dans la paroi (17) de l'incubateur et la diode laser (15) et le détecteur (16), de préférence une photodiode (16), sont disposés dans le bloc de matière (13), de préférence en aluminium, à une distance définie de la paroi (17).

8. Capteur de gaz TDLS selon l'une des revendications précédentes 2 à 7, dans lequel le bloc de matière (13) avec la diode laser (15) et le détecteur (16), de préférence la photodiode (16), est centré par rapport à la cellule d'absorption (3) et un ressort (14) du côté arrière du bloc de matière (13) opposé à la diode laser (15) pousse celui-ci dans un dispositif de centrale (12).

9. Capteur de gaz TDLS selon l'une des revendications précédentes, dans lequel la cellule d'absorption (3) est réalisée sous la forme d'une pièce cylindrique qui présente le réflecteur (10) à une extrémité, la fenêtre (9) à l'autre extrémité et des parois périphériques (7) munies d'évidements (11).

10. Capteur de gaz TDLS selon l'une des revendications précédentes, dans lequel le réflecteur (10) est réalisé sous la forme d'un miroir convexe, de préférence en acier inoxydable électropoli, et de préférence le détecteur (16), de préférence une photodiode (16), n'est pas disposé exactement au foyer du miroir convexe (10).

11. Capteur de gaz TDLS selon l'une des revendications précédentes, dans lequel la cellule de mesure (2) présente une autre diode laser (24) pour une mesure et une évaluation de l'oxygène au moyen de l'électronique de commande et d'évaluation (8).
